(19) Europäisches Patentamt
European Patent Office
Office européen des brevets

(11) Veröffentlichungsnummer: **0 125 621**
**B1**

(12) # EUROPÄISCHE PATENTSCHRIFT

(45) Veröffentlichungstag der Patentschrift: **23.09.87**

(51) Int. Cl.⁴: **C 07 C 103/44**, C 07 C 102/00

(21) Anmeldenummer: **84105234.3**

(22) Anmeldetag: **09.05.84**

(54) **Eintopfverfahren zur Herstellung von kernsubstituierten N-Alkylanilinen.**

(30) Priorität: **13.05.83 DE 3317470**

(43) Veröffentlichungstag der Anmeldung: **21.11.84 Patentblatt 84/47**

(45) Bekanntmachung des Hinweises auf die Patenterteilung: **23.09.87 Patentblatt 87/39**

(84) Benannte Vertragsstaaten: **CH DE FR GB IT LI**

(56) Entgegenhaltungen:
**DE-A-2 745 552**
**FR-A-2 093 718**

(73) Patentinhaber: **HOECHST AKTIENGESELLSCHAFT, Postfach 80 03 20, D-6230 Frankfurt am Main 80 (DE)**

(72) Erfinder: **Sommer, Karl, Dr., Thewaltstrasse 6, D-6240 Königstein/Taunus (DE)**
Erfinder: **Schickfluss, Rudolf, Dr., Luisenstrasse 37, D-6233 Kelkheim (Taunus) (DE)**

**EP 0 125 621 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Gegenstand der vorliegenden Erfindung ist ein Verfahren zur Herstellung von kernsubstituierten N-Alkylanilinen, die ohne Zwischenisolierung durch reduktive Alkylierung von kernsubstituierten Nitrobenzolen gewonnen werden (Eintopfverfahren).

Es wurde gefunden, daß man kernsubstituierte N-Alkylaniline der allgemeinen Formel (1)

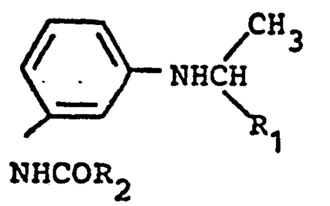

(1)

in welcher $R_1$ und $R_2$ die Methyl- oder die Äthylgruppe bedeuten, in einem Eintopfverfahren in guten Ausbeuten umweltfreundlich herstellen kann, indem man m-Nitroanilin mit einem Acylierungsmittel, welches den Acylrest -CO-$R_2$ enthält und abgibt, in einem Überschuß einer aliphatischen Carbonylverbindung der allgemeinen Formel (2)

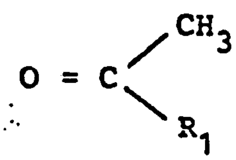

(2)

in welcher $R_1$ die weiter oben angegebene Bedeutung hat, acyliert und die entstandene Verbindung der allgemeinen Formel (3)

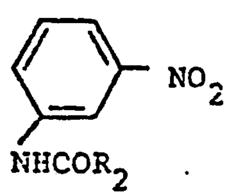

(3)

in welcher $R_2$ die oben angegebene Bedeutung hat, ohne Zwischenisolierung in Gegenwart eines sauren Reaktionsbeschleunigers und eines Nickel-Katalysators bei Temperaturen von 120 - 160°C, vorzugswoise bei 130 - 150°C, und bei einem Wasserstoffdruck von 20 - 150 bar, vorzugsweise von 50 - 100 bar, reduktiv zu einer Verbindung der allgemeinen Formel (1) alkyliert.

Die Reaktionszeit im Autoklav beträgt zwischen 4 und 7 Stunden.

Als Acylierungsmittel kommen Essigsäure- oder Propionsäurechlorid, vorzugsweise Essigsäure- oder Propionsäureanhydrid in Frage.

Als saure Reaktionsbeschleuniger können niedere gesättigte Fettsäuren, wie beispielsweise Ameisensäure, Essigsäure oder Propionsäure, oder Benzolsulfonsäure oder eine Toluolsulfonsäure eingesetzt werden. Verwendet man beim erfindungsgemäßen Verfahren Essigsäureanhydrid oder Propionsäureanhydrid als Acylierungsmittel, so wird bei der Acylierungsreaktion Essigsäure bzw. Propionsäure freigesetzt, die als saure Reaktionsbeschleuniger wirken, so daß sich in diesen Fällen der gesondere Zusatz eines sauren Reaktionsbeschleunigers erübrigt.

Geeignete Nickelkatalysatoren sind vorzugsweise Nickelkatalysatoren auf Trägern (Kohle oder Aluminiumoxid) mit etwa 50 %igem Nickelanteil.

Nach beendeter Reaktion wird der Katalysator durch Filtration abgetrennt und das Reaktionsgemisch der Vakuumdestillation unterworfen. Die zurückbleibende, etwa 70 %ige Schmelze kann als solche sofort zum Farbstoff weiterverarbeitet oder durch Zusatz von Wasser oder wäßrigen Salzlösungen zur Schmelze als Verbindung der Formel (1) abgeschiedon werden.

Die kernsubstituierten N-Alkylaniline der genannten Formel (1) lassen sich aus dem einfachen m-Nitro-anilin über die Reaktionsstufen der Acylierung, Reduktion und reduktiven Alkylierung in einem Eintopfverfahren ohne Zwischenisolierung herstellen, wobei die Ausbeute über die 3 Stufen hinweg etwa 85 % beträgt. Das einfache und wirtschaftliche Verfahren erlaubt eine umweltfreundliche Herstellung der beanspruchten Kupplungskomponenten ohne die Verwendung von cancerogenen Alkylierungsmitteln wie Diäthylsulfat, Äthylenoxid oder Acrylnitril.

In der DE-OS 2 745 552 wird ein Verfahren zur Herstellung von kernsubstituierten N-Alkylanilinen, welche sich strukturell von den nach dem erfindungsgemäßen Verfahren erhältlichen dadurch unterscheiden, daß sie unverzweigte aliphatische Substituenten am Anilinstickstoff und einen zusätzlichen Substituenten in o-Stellung zur substituierten Aminogruppe tragen, beschrieben, wobei die Nitrobenzolverbindungen mit einem Aldehyd in Gegenwart von Wasserstoff und Hydrierkatalysatoren in einem polaren, inerten Lösungsmittel zwischen 20 und 100°C umgesetzt werden. Gegenüber diesem bekannten Verfahren hat das erfindungsgemäße Verfahren den Vorteil, daß die reduktive Alkylierung auch mit den träger reagierenden Ketonen möglich ist, die hierbei gleichzeitig als Lösungsmittel und als Reaktionskomponente dienen.

**Beispiel 1**

In eine Lösung von 138 Gewichtsteilen m-Nitro-anilin in 800 Volumenteilen Methyläthylketon werden 107 Gewichtsteile Essigsäureanhydrid bei etwa 50°C zugetropft. Anschließend wird 2 Stunden bei dieser Temperatur nachgerührt.

Die so erhaltene Suspension wird in einem 2-Liter-Autoklav mit 20 Gewichtsteilen eines Nickelkatalysators nach Spülen mit Stickstoff 6 Stunden bei 135°C und bei einem Wasserstoffdruck von etwa 100 bar reduktiv alkyliert.

Die erhaltene Reaktionslösung wird vom Katalysator abgesaugt und das Lösungsmittel unter vermindertem Druck abgezogen. Die auf diese Weise erhaltene Schmelze, fast ausschließlich aus 1-Isobutylamino-3-acetylaminobenzol (Fp. 66°C) bestehend, kann durch Lösen in verdünnter Schwefelsäure direkt als Kupplungskomponente zur Herstellung von Azofarbstoffen verwendet werden.

**Beispiel 2**

Tropft man in eine Lösung von 138 Gewichtsteilen m-Nitroanilin in 800 Volumenteilen Aceton 137 Gewichtsteile Propionsäureanhydrid bei etwa 50°C zu und verfährt im übrigen wie in Beispiel 1 beschrieben, so erhält man eine Schmelze, die fast ausschließlich aus 1-Isopropylamino-3-propionylaminobenzol (Fp. 67°C) besteht, und die wiederum durch Lösen in verdünnter Schwefelsäure unmittelbar als Kupplungskomponente zur Herstellung von Azofarbstoffen eingesetzt werden kann.

**Patentansprüche**

1. Verfahren zur Herstellung von kernsubstituierten N-Alkylanilinen der allgemeinen Formel (1)

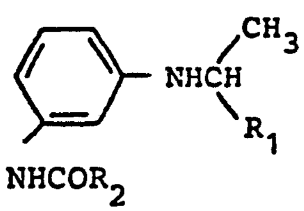

(1)

in welcher $R_1$ und $R_2$ die Methyl- oder die Äthylgruppe bedeuten in einem Eintopfverfahren, dadurch gekennzeichnet, daß man m-Nitro-anilin mit einem Acylierungsmittel,welches den Acylrest -CO-$R_2$ enthält und abgibt, im Überschuß einer aliphatischen Carbonylverbindung der allgemeinen Formel (2)

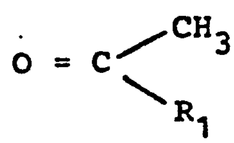

(2)

in welcher $R_1$ die vorstehend genannte Bedeutung hat, acyliert und die entstandene Verbindung der allgemeinen Formel (3)

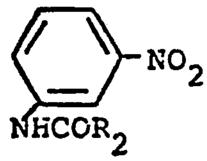

(3)

in welcher $R_2$ die vorstehend genannte Bedeutung hat, ohne Zwischenisolierung in Gegenwart eines sauren Reaktionsbeschleunigers und eines Nickelkatalysators bei Temperaturen von 120 - 160°C und bei einem Wasseistoffdruck von 20 - 150 bar reduktiv alkyliert.

2. Verfahren nach Anspruch 1, dadurch gekennzeichnet, daß man als Acylierungsmittel Essigsäureanhydrid oder Propionsäureanhydrid einsetzt.

3. Verfahren nach Anspruch 1 oder 2, dadurch gekennzeichnet, daß man als sauren Reaktionsbeschleuniger eine niedere gesättigte Fettsäure verwendet.

4. Verfahren nach Ansprüchen 1 - 3, dadurch gekennzeichnet, daß man bei Temperaturen von 130 - 150°C und bei einem Wasserstoffdruck von 50 bis 100 bar reduktiv alkyliert.

5. Verfahren nach Ansprüchen 1 - 4, dadurch gekennzeichnet, daß man als Nickelkatalysator einen Nickelkatalysator auf Trägern mit etwa 50 %igem Nickelanteil einsetzt.

## Claims

1. A process for preparing nucleus-substituted H-alkylanilines of the general formula (1)

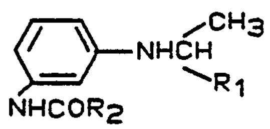

(1)

in which $R_1$ and $R_2$ each denote the methyl or ethyl group, in a single vessel, which comprises acylating m-nitroaniline in an excess of an aliphatic carbonyl compound of the formula (2)

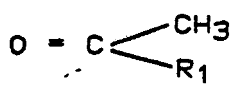

(2)

in which $R_1$ is as defined above, with an acylating agent which contains the -CO-$R_2$ acyl radical - which is transferred - and reductively alkylating the resulting compound of the formula (3)

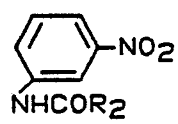

(3)

in which $R_2$ is as defined above, at temperatures of 120-160°C under a hydrogen pressure of 20-150 bar without intermediate isolation through the presence of an acid reaction promoter and of a nickel catalyst.

2. The process as claimed in claim 1, wherein as acylating agent there is used acetic anhydride or propionic anhydride.

3. The process as claimed in claim 1 or 2, wherein as the acid reaction promoter a lower saturated fatty acid is used.

4. The process as claimed in claims 1-3, wherein the reductive alkylation is carried out at temperatures of 130-150°C under a hydrogen pressure of 50 to 100 bar.

5. The process as claimed in claims 1-4, wherein as nickel catalyst there is used a nickel catalyst on carriers having an approximately 50 % nickel content.

## Revendications

1. Procédé pour préparer des N-alkylanilines substituées sur le noyau qui répondent à la formule générale 1:

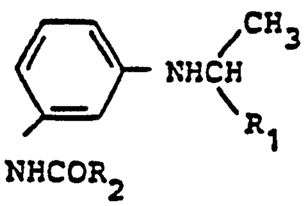

(1)

dans laquelle $R_1$ et $R_2$ représentent chacun un radical méthyle ou éthyle, par un mode opératoire en un seul récipient, procédé caractérisé en ce qu'on acyle la m-nitroaniline au moyen d'un agent d'acylation contenant et cédant le radical acyle $-CO-R_2$, dans un excès d'un composé carbonylique aliphatique répondant à la formule générale 2:

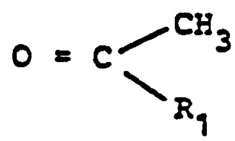

(2)

dans laquelle $R_1$ a la signification indiquée ci-dessus, et on alkyle avec réduction le composé formé qui répond à la formule générale 3:

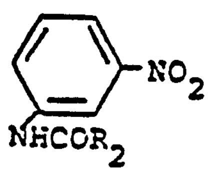

(3)

dans laquelle $R_2$ a la signification indiquée ci-dessus, sans l'isoler intermédiairement, en présence d'un accélérateur de réaction acide et d'un catalyseur au nickel, à une température de 120 à 160° C, et sous une pression d'hydrogène de 20 à 150 bar.

2. Procédé selon la revendication 1 caractérisé en ce qu'on utilise, comme agent d'acylation, l'anhydride acétique ou l'anhydride propionique.

3. Procédé selon l'une des revendications 1 et 2 caractérisé en ce qu'on utilise, comme accélérateur de réaction acide, un acide gras saturé inférieur.

4. Procédé selon l'une guelconque des revendications 1 à 3, caractérisé en ce qu'on effectue l'alkylation réductrice à une température de 130 à 150° C et sous une pression d'hydrogène de 50 à 100 bar.

5. Procédé selon l'une quelcongue des revendications 1 à 4, caractérisé en ce qu'on utilise, comme catalyseur au nickel, un catalyseur au nickel sur support dont la teneur en nickel est d'environ 50 %.